# EUROPEAN PATENT APPLICATION

(11) **EP 2 075 581 A1**
(43) Date of publication of application: **01.07.2009**
(21) Application number: 07806648.7
(22) Date of filing: 04.09.2007
(51) Int. Cl.: G01N 33/533, C12Q 1/68, G01N 21/64, G01N 21/78, G01N 33/58

(54) **SEMICONDUCTOR NANOPARTICLE AGGREGATE, PROCESS FOR PRODUCING THE SEMICONDUCTOR NANOPARTICLE AGGREGATE, AND BIOLOGICAL SUBSTANCE LABELING AGENT USING THE SEMICONDUCTOR NANOPARTICLE AGGREGATE**

(30) Priority: 14.09.2006 JP 2006249207
(71) Applicant: Konica Minolta Medical & Graphic, Inc., Hino-shi, Tokyo 191-8511 (JP)
(72) Inventor: FURUSAWA, Naoko, Hino-shi, Tokyo 191-8511 (JP); TSUKADA, Kazuya, Hino-shi, Tokyo 191-8511 (JP); OKADA, Hisatake, Hino-shi, Tokyo 191-8511 (JP)
(74) Representative: Alton, Andrew
(86) International application number: PCT/JP2007/067181
(87) International publication number: WO 2008/032599

(57) **Abstract**

This invention provides a semiconductor nanoparticle aggregate comprising three or more types of semiconductor nanoparticles, which are different from each other in diameter, have a narrow particle size distribution, and are different from each other in maximum luminous wavelength of an emission spectrum in a wavelength region of 380 nm to 650 nm, a process for producing the semiconductor nanoparticle aggregate, and a biological substance labeling agent utilizing the semiconductor nanoparticle aggregate. The semiconductor nanoparticle aggregate comprises three or more types of semiconductor nanoparticles which have an identical chemical composition, are different from each other in particle diameter and fall within a particle diameter range of 1.8 to 4 nm and are different from each other in maximum luminous wavelength of an emission spectrum in a wavelength range of 380 to 650 nm. The semiconductor nanoparticle aggregate is **characterized in that** the difference in maximum luminous wavelength among three or more types of semiconductor nanoparticles constituting the semiconductor nanoparticle aggregate is in the range of 20 to 100 nm.

## Description

### TECHNICAL FIELD

The present invention relates to a semiconductor nanoparticle aggregate, a method for producing the same, and a biological substance labeling agent utilizing the same.

### BACKGROUND

Recent advancement in nanotechnology indicates the possibility that nanoparticles can be used in detection, diagnosis, sensing, and other applications. Further, over recent years, nanoparticle complexes, which interact with biological systems, have attracted wide attention in the biological and medical fields. These complexes are thought to be promising as novel intravascular probes for both sensing (e.g., imaging) and medical treatment purposes (e.g., drug delivery).

Incidentally, a material, which is a semiconductor material of nanometer size, producing a quantum confinement effect is referred to as "a quantum dot." Such a dot is a tiny agglomerate of at most ten-odd nm composed of several hundreds to several thousands of aggregated semiconductor atoms, and emits energy corresponding to the energy band gap of the quantum dot when reaching an energy excitation state via absorption of light from an excitation source. Accordingly, when the size or material composition of the quantum dot is adjusted, the energy band gap can be controlled, whereby energy can be utilized at various wavelength bands.

Further, quantum dots are **characterized in that** emission wavelength can be controlled by changing the particle diameters even when the compositions are the same, and are attracting attention in view of stability and brightness of emitting light, compared to organic fluorescent dyes which are conventional technologies.

Currently, CdSe particles are commonly used as fluorescent materials, realizing multicolor emission via size variations. However, the size of visible light emission is 5 nm - ten-odd nm, which is larger than those of biological molecules, whereby movement of the biological molecules may be hindered, resulting in unfavorable observation. Therefore, it has been desired that multicolor emission at 4 nm or less is realized.

With the same composition, there can be realized such a large advantage that the specific gravities of quantum dots become the same for each of the colors. Combined use of nanoparticles and fluorescent dye molecules of different composition tends to result in separation in a staining solution, which has produced the problem that handling is very cumbersome (for example, refer to Patent Documents 1 and 2).
Patent Document 1: Japanese Translation of PCT International Application Publication No. 2003-524147
Patent Document 2: Unexamined Japanese Patent Application Publication (hereinafter referred to as JP-A) No. 2005-172429

### DISCLOSURE OF THE INVENTION

### PROBLEMS TO BE SOLVED BY THE INVENTION

In view of the above problems, the present invention was achieved. An object of the present invention is to provide a semiconductor nanoparticle aggregate composed of at least 3 types of semiconductor nanoparticles, having different particle diameters, narrow particle diameter distribution, and different maximum emission wavelengths in the emission spectra in a wavelength region of 380 nm - 650 nm, and a production method thereof, and further to provide a biological substance labeling agent utilizing the semiconductor nanoparticle aggregate.

### MEANS TO SOLVE THE PROBLEMS

The above problems can be solved via the following means:
1. In a semiconductor nanoparticle aggregate composed of at least 3 types of semiconductor nanoparticles having the same chemical composition, different particle diameters in a particle diameter range of 1.8 - 4 nm, and different maximum emission wavelengths in the emission spectra in a wavelength region of 380 nm - 650 nm, a semiconductor nanoparticle aggregate wherein the difference of the maximum emission wavelengths of at least 3 types of semiconductor nanoparticles constituting the semiconductor nanoparticle aggregate falls within the range of 20 - 100 nm.
2. In the semiconductor nanoparticle aggregate described in means 1, a semiconductor nanoparticle aggregate wherein at least 1 type of semiconductor nanoparticle constituting the semiconductor nanoparticle aggregate is formed with a compound featuring a chemical composition having a bulk crystal band gap of at most 1.3 eV.
3. In the semiconductor nanoparticle aggregate described in means 1 or 2, a semiconductor nanoparticle aggregate wherein the half-value width of an emission band of the maximum emission wavelength in the emission spectrum of at least 1 type of semiconductor nanoparticle constituting the semiconductor nanoparticle aggregate is at most 50 nm.
4. In the semiconductor nanoparticle aggregate described in any of means 1 - 3, a semiconductor nanoparticle aggregate wherein the half-value width of an emission band of the maximum emission wavelength in the emission spectrum of at least 1 type of semiconductor nanoparticle constituting the semiconductor nanoparticle aggregate is at most 20 nm.
5. In the semiconductor nanoparticle aggregate described in any of means 1 - 4, a semiconductor nanoparticle aggregate wherein semiconductor nanoparticles constituting the semiconductor nanoparticle aggregate are classified particles.
6. The semiconductor nanoparticle aggregate, described in mean 5, wherein the classification is carried out using a high-performance liquid chromatography method.
7. In a method for producing the semiconductor nanoparticle aggregate described in any of means 1 - 4, a method for producing a semiconductor nanoparticle aggregate wherein a classification process of semiconductor nanoparticles is incorporated.
8. The method for producing a semiconductor nanoparticle aggregate, described in means 7, wherein a high-performance liquid chromatography method is used in the classification process.
9. A biological substance labeling agent wherein the semiconductor nanoparticle aggregate described in any of means 1 - 6 and a molecule labeling substance are bonded via an organic molecule.

### EFFECTS OF THE INVENTION

According to the above means of the present invention, there can be provided a semiconductor nanoparticle aggregate composed of at least 3 types of semiconductor nanoparticles, having different particle diameters, narrow particle diameter distribution, and different maximum emission wavelengths in the emission spectra in a wavelength region of 380 nm - 650 nm, and a production method thereof, and further to provide a biological substance labeling agent utilizing the semiconductor nanoparticle aggregate.

### DESCRIPTION OF THE PREFERRED EMBODIMENT

In the present invention according to above means 1 - 6, a semiconductor nanoparticle aggregate is **characterized in that** in a semiconductor nanoparticle aggregate composed of at least 3 types of semiconductor nanoparticles having the same chemical composition, different particle diameters in a particle diameter range of 1.8 - 4 nm, and different maximum emission wavelengths in the emission spectra in a wavelength region of 380 nm - 650 nm, the difference of the maximum emission wavelengths of at least 3 types of semiconductor nanoparticles constituting the semiconductor nanoparticle aggregate falls within the range of 20 - 100 nm.

This semiconductor nanoparticle aggregate is intended to be specifically used as a labeling compound. Particles of different emission wavelength each are separately allowed to bond to different antibodies or DNAs of different sequence, and then mixed with a subject to be examined or injected into a cell. Thereby, different subjects can simultaneously be detected, resulting in shortening of examination duration. In cell imaging, there are such advantages that a plurality of subjects can simultaneously be stained and also observation accuracy is enhanced.

The present invention and the components thereof will now be detailed.

### (Semiconductor Nanoparticle Aggregate)

"Semiconductor nanoparticle aggregate" according to the present invention refers to a solution incorporating nanoparticles, a sheet incorporating dispersed nanoparticles, or a powder composed of nanoparticles.

With regard to such quantum dots having a small Bohr radius and a small visible light emission size, there is produced the problem that it is difficult to separately produce each size via controlling of synthesis conditions.

The semiconductor nanoparticle aggregate of the present invention is **characterized in that** in a semiconductor nanoparticle aggregate composed of at least 3 types of semiconductor nanoparticles having the same chemical composition, different particle diameters in a particle diameter range of 1.8 - 4 nm, and different maximum emission wavelengths in the emission spectra in a wavelength region of 380 nm - 650 nm, the difference of the maximum emission wavelengths of at least 3 types of semiconductor nanoparticles constituting the semiconductor nanoparticle aggregate falls within the range of 20 - 100 nm.

Further, in the semiconductor nanoparticle aggregate of the present invention, at least 1 type of semiconductor nanoparticle constituting the semiconductor nanoparticle aggregate is preferably formed with a compound featuring a chemical composition having a bulk crystal band gap of at most 1.3 eV.

Still further, the half-value width of an emission band of the maximum emission wavelength in the emission spectrum of at least 1 type of semiconductor nanoparticle constituting the semiconductor nanoparticle aggregate is preferably at most 50 nm. The half-value width is more preferably at most 20 nm.

Incidentally, to realize the above half-value width, semiconductor nanoparticles constituting the semiconductor nanoparticle aggregate of the present invention are preferably classified particles. Herein, "classification" refers to an operation to distinguish particle powders based on particle diameter, density, or shape. In the present invention, an operation to distinguish specifically based on particle diameter (size) is referred to.

As a classification method, any conventionally known methods can be used, but classification is preferably carried out via a high-performance liquid chromatography (HPCL) method, for example, a size-exclusion chromatography method as one embodiment thereof. This method is specifically described in examples to be described later.

### (Semiconductor Nanoparticles)

With regard to semiconductor nanoparticles constituting the semiconductor nanoparticle aggregate of the present invention, the present inventors conducted diligent investigations and found that in order to decrease the size of semiconductor nanoparticles (hereinafter also referred to as "quantum dots") according to the present invention, it was necessary to decrease exciton-Bohr radius, and a requirement for realizing the above was that the bulk band gap value of a semiconductor composition forming semiconductor nanoparticles (quantum dots) was at most 1.3 eV.

When prepared using a composition satisfying this requirement, quantum dots of at most 5 nm make it possible to cover the visible light region.

Semiconductor compositions falling within this range include Si, Ge, GaSb, InAs, and InSb.

Further, in semiconductors of groups III - V, of A_{xB(1 - x)}C (A and B are one selected from Al, Ga, and In; and C is one selected from N, P, As, and Sb), those with an x value adjusted so as for the band gap value to be at most 1.3 eV via combination can preferably be used.

When the particle diameter becomes less than 1.8 nm, no crystal structure is formed, whereby no functions as quantum dots are fulfilled.

### <Forming Materials of Semiconductor Nanoparticles>

Forming materials of semiconductor nanoparticles according to the present invention are preferably those satisfying the above requirement, and can be formed using various types of semiconductor materials. For example, semiconductor compounds of group IV, groups II - VI, and groups III - V in the periodic table of the elements can be used.

Of semiconductors of groups II - VI, there are specifically listed MgS, MgSe, MgSe, MgTe, CaS, CaSe, CaTe, SrS, SrSe, SrTe, BaS, BaSe, BaTe, ZnS, ZnSe, ZnTe, CdS, CdSe, HgS, HgSe, and HgTe.

Of semiconductors of groups III - V, there are preferable GaAs, GaN, GaPGaSb, InGaAs, InP, InN, InSb, InAs, AlAs, AlP, AlSb, and AlS.

Of semiconductors of group IV, Ge, Pb, and Si are specifically suitable.

In the present invention, to apply a semiconductor nanoparticle aggregate to a biological substance labeling agent, a semiconductor nanoparticle is preferably formed as a particle having a core/shell structure. In this case, such a semiconductor nanoparticle is a so-called core/shell type semiconductor particle having a core/shell structure which is structured of a core particle composed of a semiconductor nanoparticle and a shell layer covering the core particle. The core particle and the shell layer preferably each differ in chemical composition.

Further, the average particle diameter of the core particle is preferably 1 - 4 nm, more preferably 1.8 - 4 nm from the viewpoint of exhibiting quantum size effects. Incidentally, even the average particle diameter of a core/shell type semiconductor particle, to which a shell is added, is also preferably at most 4 nm, since labeling and dynamic imaging for one molecule of a living body can be carried out.

"Average particle diameter" described above can be determined via a method employing high-resolution transmission electron microscope (TEM) photographic observation, or a laser scattering method.

The core particle and the shell layer will now be described.

### <Core Particle>

As semiconductor materials used for a core particle, various types of semiconductor materials can be employed. There are specifically listed, for example, MgS, MgSe, MgTe, CaS, CaSe, CaTe, SrS, SrSe, SrTe, BaS, BaTe, ZnS, ZnSe, ZnTe, CdS, CdSe, CdTe, GaAs, GaP, GaSb, InGaAs, InP, InN, InSb, InAs, AlAs, AlP, AlSb, AlS, PbS, PbSe, Ge, and Se, or mixtures thereof. In the present invention, a specifically preferable semiconductor material is Si.

Incidentally, an ultra-small amount of a dope material such as Ga may be incorporated, if appropriate.

### <Shell Layer>

As semiconductor materials used for a shell, various types of semiconductor materials can be employed. There are specifically listed, for example, ZnO, ZnS, ZnSe, ZnTe, CdO, CdS, CdSe, CdTe, MgS, MgSe, GaS, GaN, GaP, GaAs, GaSb, InAs, InN, InP, InSb, AlAs, AlN, AlP, and AlSb, or mixtures thereof.

Incidentally, preferable shell materials include semiconductive materials exhibiting a higher band gap than a semiconductive nanocrystal core. In addition to having higher band gap energy than a semiconductive fine particle crystal core, materials suitable for a shell needs to exhibit excellent conductivity and atomic valence band offset with respect to a core semiconductive nanocrystal. Therefore, the conductive band is preferably higher than that of the core semiconductive nanocrystal, and the atomic valence band is preferably lower than that of the core semiconductive nanocrystal. For a semiconductive nanocrystal core emitting energy in the visible region (e.g., Si, Ge, and GaP) or in the near-infrared region (e.g., InP, InN, PbS, and PbSe), materials having band gap energy in the UV region can be used. Specific examples include, for example, ZnS, GaN, and magnesium chalcogenides (e.g., MgS, MgSe, and MgTe).

In the present invention, specifically preferable semiconductor materials are SiO₂ and ZnS.

### <Production Method of Semiconductor Nanoparticles>

To produce the semiconductor nanoparticle of the present invention, various types of methods conventionally known in the art can be employed.

Production methods employing a liquid phase method include a coprecipitation method, a sol-gel method, a homogeneous precipitation method, and a reduction method, all of which are precipitation methods. In addition, a reverse-micelle method and a supercritical hydrothermal synthesis method are excellent methods to produce nanoparticles (for example, refer to JP-A Nos. 2002-322468, 2005-239775, 10-310770, and 2000-104058).

As production methods employing a gas phase method, there are used (1) a method wherein facing raw material semiconductors are evaporated with a first high-temperature plasma generated between electrodes, followed by being passed through a second high-temperature plasma generated via electrodeless discharging under reduced pressure (for example, refer to JP-A No. 6-279015), and (2) a method wherein nanoparticles are separated and removed from an anode composed of a raw material semiconductor via electrochemical etching (for example, refer to Japanese Translation of PCT International Application Publication No. 2003-515459) or a laser abrasion method (for example, refer to JP-A No. 2004-356163). Further, there is preferably used a method wherein a raw material gas is subjected to a gas phase reaction under reduced pressure to synthesize powder incorporating particles.

As a method for producing the semiconductor nanoparticle of the present invention, a production method specifically employing a liquid phase method is preferable.

Incidentally, to realize uniformity of the particle diameter and the emission intensity of a semiconductor nanoparticle according to the present invention, it is necessary to form a semiconductor nanoparticle having less lattice defects and exhibiting enhanced crystallinity by optimizing the purity of a raw material, the synthesis concentration, the synthesis temperature and duration, and the anneal temperature and duration after particle formation.

### (Biological Substance Labeling Agent and Bioimaging)

The semiconductor nanoparticle aggregate of the present invention is adaptable to a biological substance fluorescent labeling agent. Further, when added to a living cell or a living body having a target (trace) substance, a biological substance labeling agent according to the present invention is bonded or adsorbed to the target substance. Thereafter, the resulting bonded or adsorbed body is irradiated with excitation light of a predetermined wavelength and then fluorescence of a specific wavelength emitted from a fluorescent semiconductor fine particle based on the excitation light is detected. Thereby, fluorescent dynamic imaging of the target (trace) substance can be carried out. Namely, the biological substance labeling agent of the present invention can be utilized in a bioimaging method (a technological method to visualize biological molecules constituting a biological substance and dynamic phenomena thereof).

### [Hydrophilization of Semiconductor Nanoparticle Aggregate]

The surface of the above semiconductor nanoparticle aggregate is usually hydrophobic. Therefore, for example, in the case of use as a biological substance labeling agent, the aggregate as such exhibits poor water dispersibility, resulting in agglomerated particles which are problematic, whereby the shell surface of a core/shell type semiconductor nanoparticle is preferably hydrophilized.

A method of hydrophilization includes, for example, a method wherein lipophilic groups on the surface are removed with pyridine and then a surface modifier is allowed to bond to the particle surface in at least one manner of a chemical and a physical manner. As the surface modifier, those containing a carboxyl group or an amino group as a hydrophilic group are preferably used. There are specifically listed, for example, mercaptopropionic acid, mercaptoundecanoic acid, and aminopropanethiol. Specifically, for example, 10⁻⁵ g of a Ge/GeO₂ type nanoparticle is dispersed in 10 ml of purified water dissolving 0.2 g of mercaptoundecanoic acid and stirred at 40 °C for 10 minutes, followed by surface treatment of the shell to modify the shell surface of the inorganic nanoparticle with a carboxyl group.

### [Biological Substance Labeling Agent]

A biological substance labeling agent according to the present invention is obtained by allowing the above hydrophilized semiconductor nanoparticle to bond to a molecule labeling agent via an organic molecule.

### [Molecule Labeling Agent]

A biological substance labeling agent according to the present invention can label a biological substance in such a manner that a molecule labeling substance is specifically allowed to bond to and/or react with the targeted biological substance.

Such a molecule labeling substance includes, for example, a nucleotide chain, an antibody, an antigen, and cyclodextrin.

### <Organic Molecule>

In a biological substance labeling agent according to the present invention, a hydrophilized semiconductor nanoparticle is bonded to a molecule labeling agent via an organic molecule. The organic molecule is not specifically limited, if being an organic molecule allowing a semiconductor nanoparticle and a molecule labeling agent to join together. For example, of proteins, there are preferably used albumin, myoglobin, and casein, as well as avidin, a type of protein, combined with biotin. The above bonding embodiment is not specifically limited, including covalent bonding, ionic bonding, hydrogen bonding, coordination bonding, physical adsorption, and chemical adsorption. From the viewpoint of bonding stability, bonding featuring a strong bonding force such as covalent bonding is preferable.

Specifically, when a semiconductor nanoparticle is hydrophilized with mercaptoundecanoic acid, avidin and biotin can be used as organic molecules. In this case, the carboxyl group of the hydrophilized nanoparticle is preferably covalent bonded to avidin, which is then selectively bonded to biotin. Thereafter, the biotin is further bonded to a biological substance labeling agent to give a biological substance labeling agent.

### EXAMPLES

The present invention will now be detailed with reference to examples that by no means limit the scope of the present invention.

### Example 1

A mixed gas of monosilane gas and argon gas was introduced at 0.7 l/minute and 19.3 l/minute, respectively, into a reaction pipe made of quartz glass kept at 820 °C and 50 kPa to perform reaction for 0.5 hour. Then, powder was formed at the bottom of the reaction pipe. The reaction pipe was cooled, and then 1 mg of the powder was mixed with 100 ml of decane to disperse a Si semiconductor nanoparticle in the decane via ultrasonic dispersion. The resulting solution is designated as sample 1.

This nanoparticle was classified via size-exclusion chromatography. A reversed-phase ODS (octadecylsilyl group C-18) column was used as the column. Acetonitrile was used as the mobile phase and allowed to pass through the column at a flow rate of 0.1 ml/minute. An absorption spectrum of 365 nm was measured over time and then absorption was confirmed in the range of 3 minutes - 30 minutes.

Sampling was carried out at 6 time points of 6, 10, 15, 20, 25, and 30 minutes each for 30 seconds to prepare samples 1-1, 1-2, 1-3, 1-4, 1-5, and 1-6.

Using a high-resolution transmission electron microscope (TEM) photography, average particle diameter and particle diameter distribution were measured with respect to 100 semiconductor nanoparticles.

Herein, the following relationship is defined: average particle diameter distribution = the number percent of particles falling within ±50% of the average particle diameter.

Using Hitachi spectrophotometer F7000, the emission spectrum of each of 6 types of the semiconductor nanoparticles was measured at an excitation wavelength of 365 nm to carry out comparison among maximum emission wavelength intensities.

XPS measurement verified that the composition was Si. The band gap value of the bulk Si is 1.12 eV.

**[Table 1]**

| | Average Particle Diameter | Particle Diameter Distribution | Maximum Emission Wavelength | Half-value Width |
|---|---|---|---|---|
| Sample 1-1 | 3.8 nm | 2.3% | 683 nm | 15 nm |
| Sample 1-2 | 3.4 nm | 3.5% | 620 nm | 18 nm |
| Sample 1-3 | 3.0 nm | 1.6% | 580 nm | 16 nm |
| Sample 1-4 | 2.5 nm | 4.2% | 465 nm | 16 nm |
| Sample 1-5 | 2.1 nm | 5.2% | 405 nm | 17 nm |
| Sample 1-6 | 1.8 nm | 6.3% | 378 nm | 19 nm |

A mixture of at least 3 types selected from above samples 1-1 - 1-6 is designated as the semiconductor nanoparticle aggregate of the present invention.

### Comparative Example 1

A three-necked flask was charged with trioctylphosphine selenide (6.15 g, 7.5 mM) and trioctylphosphine oxide (19.33 g, 50 mM), followed by temperature adjustment at 300 °C. During vigorous stirring, cadmium acetate dihydrate (1.97 g, 7.5 mM) was rapidly introduced, and thereafter stirring was carried out for 5 hours while the temperature was kept at 250 °C.

The reaction system was cooled to room temperature, and then ethanol was added thereto, followed by centrifugal separation. The supernatant liquid was removed and the deposit was redispersed in decane to obtain sample 2 which was a CdSe nanoparticle solution.

Sample 2 was classified via size-exclusion chromatography. A reversed-phase ODS (octadecylsilyl group C-18) column was used as the column. Acetonitrile was used as the mobile phase and allowed to pass through the column at a flow rate of 0.05 ml/minute. An absorption spectrum of 365 nm was measured over time and then absorption was confirmed in the range of 1 minute - 20 minutes.

Sampling was carried out at 3 time points of 10, 15, and 20 minutes each for 15 seconds to prepare samples 2-1, 2-2, and 2-3.

Using a high-resolution transmission electron microscope (TEM) photography, average particle diameter and particle diameter distribution were measured with respect to 100 nanoparticles.

Herein, the following relationship is defined: average particle diameter distribution = the number percent of particles falling within ±50% of the average particle diameter.

Using Hitachi spectrophotometer F7000, the emission spectrum of each of 3 types of the semiconductor nanoparticles was measured at an excitation wavelength of 365 nm to carry out comparison among maximum emission wavelength intensities.

XPS measurement verified that the composition was CdSe. The band gap value of the bulk CdSe is 1.74 eV.

**[Table 2]**

| | Average Particle Diameter | Particle Diameter Distribution | Maximum Emission Wavelength | Half-value Width |
|---|---|---|---|---|
| Sample 2-1 | 7.2 nm | 2.1% | 621 nm | 13 nm |
| Sample 2-2 | 4.8 nm | 1.9% | 528 nm | 11 nm |
| Sample 2-3 | 3.2 nm | 2.7% | 394 nm | 16 nm |

The above result shows that CdSe, which is outside of the scope of the present invention, can exhibit no visible light range emission when the size thereof is at most 4 nm.

### Example 2

There was redispersed 1 x 10⁻⁵ g of each of various types of the Si semiconductor nanoparticles prepared in example 1 in 10 ml of purified water dissolving 0.2 g of mercaptoundecanoic acid, followed by being stirred at 40 °C for 10 minutes to give various types of surface-hydrophilized nanoparticles.

Thereafter, 25 mg of avidin was added to a solution of each of various types of the surface-hydrophilized nanoparticles, followed by being stirred at 40 °C for 10 minutes to prepare avidin-conjugated nanoparticles.

A biotinylated oligonucleotide having a known base sequence was mixed with an obtained avidin-conjugated nanoparticle solution while stirring to prepare a nanoparticle-labeled oligonucleotide.

The above labeled oligonucleotide was dropped onto a DNA chip tightly holding oligonucleotides having various base sequences, followed by washing. Then, it was shown that of these, only the spot of an oligonucleotide, having a base sequence complementary to that of the labeled oligonucleotide, emitted light of different color based on the particle diameter of the semiconductor nanoparticle via UV irradiation.

The above result showed that oligonucleotide labeling was able to be carried out using the semiconductor nanoparticle of the present invention.

## Claims

1. A semiconductor nanoparticle aggregate comprising at least 3 types of semiconductor nanoparticles each having the same chemical composition, a different average particle diameter in a range of 1.8 - 4 nm, and a different maximum emission wavelength in emission spectra in a wavelength region of 380 nm - 650 nm,
wherein a difference of wavelength between the maximum emission wavelengths is 20 - 100 nm.

2. The semiconductor nanoparticle aggregate of claim 1,
wherein at least 1 type of the semiconductor nanoparticles comprises a compound having a chemical composition exhibiting a bulk crystal band gap of 1.3 eV or less.

3. The semiconductor nanoparticle aggregate of claim 1 or 2, wherein a half-value width of an emission band of the maximum emission wavelength in the emission spectrum of at least 1 type of the semiconductor nanoparticle is 50 nm or less.

4. The semiconductor nanoparticle aggregate in any of claims 1 - 3, wherein the half-value width of the emission band of the maximum emission wavelength in the emission spectrum of at least 1 type of the semiconductor nanoparticle is 20 nm or less.

5. The semiconductor nanoparticle aggregate in any of claims 1 - 4, wherein the semiconductor nanoparticles are classified particles.

6. The semiconductor nanoparticle aggregate of claim 5,
wherein the classification is carried out using a high-performance liquid chromatography method.

7. A method for producing the semiconductor nanoparticle aggregate in any of claims 1 - 4, wherein a classification process of semiconductor nanoparticles is incorporated.

8. The method for producing a semiconductor nanoparticle aggregate of claim 7, wherein the high-performance liquid chromatography method is used in the classification process.

9. A biological substance labeling agent, wherein the semiconductor nanoparticle aggregate in any of claims 1 - 6 and a molecule labeling substance are bonded via an organic molecule.
